# FASCICULE DE BREVET EUROPEEN

(11) **EP 4 326 152 B1**
(45) Date de publication et mention de la délivrance du brevet: **04.11.2026**
(21) Numéro de dépôt: 22722865.7
(22) Date de dépôt: 20.04.2022
(51) Int. Cl.: A61B 5/16, A61B 5/00, G06F 3/01, G16H 50/20, A61B 5/11, A61H 5/00, A61B 3/08

(54) **SYSTEME DE TRAITEMENT DE TROUBLES NEUROVISUELS OU VESTIBULAIRES**
SYSTEM ZUR BEHANDLUNG NEUROVISUELLER ODER VESTIBULÄRER ERKRANKUNGEN
SYSTEM FOR TREATING NEUROVISUAL OR VESTIBULAR DISORDERS

(30) Priorité: 20.04.2021 FR 2104102
(43) Date de publication de la demande: 28.02.2024
(73) Titulaire: Scale-1 Portal, 78180 Montigny-Le-Bretonneux (FR); Ocular Brain, 76800 Saint-Etienne-Du-Rouvray (FR)
(72) Inventeur: ICART, Emmanuel, 78310 COIGNIERES (FR); BAESSENS, Julien, 78280 GUYANCOURT (FR); LEGRAND, Olivier, 28500 MONTREUIL (FR); BRUGULAT, Marie, 91160 CHAMPLAN (FR); GIBERT, Charlotte, 76100 ROUEN (FR)
(74) Mandataire: IP Trust
(86) Numéro de dépôt international: PCT/FR2022/050736
(87) Numéro de publication internationale: WO 2022/223924

(56) Documents cités:
- WO-A2-2018/046957
- US-A1- 2013 021 373
- US-A1- 2016 015 289
- US-A1- 2016 262 608
- US-A1- 2017 365 101
- US-A1- 2018 184 964

## Description

### DOMAINE DE l'INVENTION

La présente invention concerne un système de traitement de troubles neurovisuels ou vestibulaires.

### ETAT DE LA TECHNIQUE

Parmi les outils actuellement utilisés par les orthoptistes, on utilise des présentations d'objets avec saturation de la vision périphérique afin de favoriser l'attention visuelle. Sont également utilisées des saccades : jeu de cartes, labyrinthe, cache-cache safari, perle BAHA, loto, barrage (Attention mes yeux, mes mots), lecture hachée, discrimination figure/fond, imbroglio d'images/lettres, superposition repérage, suivi de lignes, copie et coloriage, mots mêlés, jeu du lynx, passage de la première lettre à la dernière, jeu des différences ou des 7 erreurs, jeu de discrimination, coordination oeil-main. Il existe également des méthodes d'oculométrie mettant par exemple en œuvre des techniques dites d'« eye tracking » (poursuite oculaire) pour réaliser un feedback (retour) des saccades oculaires. Les orthoptistes considèrent dans leur grande majorité que les troubles neurovisuels observés chez les enfants sont résolus après une ou plusieurs années de prise en charge [1].

Enfin, quel que soit le trouble neurovisuel, les séances actuelles de rééducation se composent en deux parties (rééducation classique puis fonctionnelle) comme pour le bilan. Par manque de matériel orthoptique adapté, les praticiens utilisent beaucoup de matériel orthophonique qu'ils détournent de leur destination première, étant donné des objectifs thérapeutiques différents de ceux fixés par les orthophonistes. Lors des rééducations fonctionnelles, les orthoptistes travaillent le plus souvent les saccades, aident les enfants à mettre en place des stratégies de balayage à l'aide de livres comme «Où est Charlie?» ou de matériel comme «attention mes yeux», et travaillent la mémoire visuelle, l'organisation spatiale (Glup, labyrinthes) et l'attention (jeu des différences). [2]

Le document US9691289 divulgue un système et procédé semblable à un jeu (gamification) qui génère de nouveaux stimuli non verbaux déclenchant des déplacements de l'attention visuelle, dans le but d'améliorer la reconnaissance fovéale et parafovéale des stimuli non verbaux et des stimuli verbaux présentés latéralement dans le champ visuel droit ou gauche. Ce procédé fait appel à un réseau neuronal perceptuel et cognitif partagé qui fait intervenir des comportements cognitifs oculomoteurs, visuo-motorisés et exécutifs sélectifs dans les deux hémisphères du cerveau.

Le document US2020/0329961 divulgue un appareil pour le dépistage, le traitement, la surveillance et/ou l'évaluation des déficiences visuelles, comprenant des moyens électroniques pour appliquer simultanément deux méthodes de traitement séparées et sans rapport entre elles aux images présentées aux yeux d'un patient : une première méthode de traitement étant appliquée à un oeil non amblyope (l'oeil ayant la meilleure vision), et une seconde méthode de traitement étant appliquée à un oeil amblyope (l'oeil plus faible, ou l'oeil déficient).

Le document KR10-2021-0000782 divulgue un jeu de réalité virtuelle et un capteur biosignal complexe basé sur celui-ci. Le jeu de réalité virtuelle comprend un eye-tracker fixé à ou installé sur un dispositif d'affichage monté sur la tête, une unité de détection configurée pour détecter et notifier un mouvement de la tête et un mouvement des yeux du patient en utilisant un capteur de mesure inertielle (capteur IMU), une unité de commande configurée pour configurer et fournir un jeu pour évaluer une fonction réflexe vestibulo-oculaire (VOR).

Le document US 2018/0184964 A1 divulgue une méthode de diagnostic de l'autisme et/ou des troubles du spectre autistique (TSA) d'un sujet, comprenant une étape pour établir des modèles d'ondes cérébrales de base du sujet en demandant au sujet d'effectuer une série de tâches et pour mesurer des ondes cérébrales pendant les tâches à l'aide d'un appareil de mesure EEG, une étape pour appliquer un stimulus lumineux ou des images aux yeux du sujet et capturer les mouvements oculaires et/ou changements dans l'expression du visage en réponse au stimulus lumineux ou aux images.

Le document US 2017/0365101 A1 divulgue un système d'affichage comprenant un visiocasque dont l'affichage en réalité augmentée est configuré pour effectuer une analyse neurologique et fournir une aide à la perception basée sur un déclencheur environnemental associé à un état neurologique. L'analyse neurologique peut inclure la détermination d'une réaction à un stimulus en recevant des données provenant du ou des capteurs dirigés vers l'intérieur, et identifier une condition neurologique associée à la réaction.

Le document WO2018/046957 A2 divulgue un système de lecture comprenant un appareil comportant un écran et un processeur configuré pour afficher une séquence de texte à l'attention un lecteur dans un processus de lecture, et au moins une ressource de traitement configurée pour obtenir des données de lecture représentant au moins une propriété du texte et/ou du processus de lecture, et pour traiter les données de lecture afin d'identifier les changements des données de lecture au fil du temps qui peuvent indiquer une détérioration visuelle ou neurologique et/ou la présence d'au moins un état chez le lecteur.

Le document US2013/021373 A1 divulgue un dispositif de visiocasque (HMD) transparent, par exemple sous la forme de lunettes, permettant de visualiser une image de réalité augmentée comprenant du texte, comme dans un livre ou un magazine électronique, un document de traitement de texte, un courrier électronique, un karaoké, un téléprompteur ou autre demande d'aide à la prise de parole en public. La présentation de texte et/ou de graphiques peut être ajustée sur la base d'entrées de capteur indiquant une direction de regard, une distance focale et/ou une métrique biologique de l'utilisateur.

Un but principal de la présente invention est de pallier le manque actuel d'outils de traitement de troubles neurovisuels adaptés à la pratique thérapeutique des orthoptistes, en proposant un nouveau système de traitement qui soit plus performant que les dispositifs actuels mis à la disposition des praticiens et thérapeutes.

### EXPOSE DE L'INVENTION

Cet objectif est atteint avec un système pour traiter des troubles neurovisuels et/ou vestibulaires présentés par un sujet humain, selon la revendication 1.

Suivant l'invention, les moyens immersifs sont commandés par les moyens de contrôle et de traitement qui exploitent les données de tracking corporel pour procurer audit sujet humain une visualisation simultanée (i) d'un environnement virtuel périphérique défilant à une première vitesse et (ii) d'un ensemble de mots d'un texte source sur un parcours virtuel (40) défilant à une seconde vitesse, et sont agencés pour procurer au sujet humain immergé une saturation de la vision périphérique, ce système de traitement comprenant en outre :
- des moyens pour stimuler un ou plusieurs des sens dudit sujet humain, en vue de perturber sa lecture du texte source,
- des moyens pour traiter les mots ou ensemble de mots prononcés et captés, de façon à délivrer des données représentatives de l'expérience de lecture du texte source par le sujet humain en relation avec des stimuli de perturbation émis par lesdits moyens de stimulation.

Les stimuli de perturbation peuvent par exemple comprendre une émission d'un signal sonore perçu par le sujet humain, une modification de caractéristiques d'affichage du texte source, ou une visualisation d'un élément de distraction dans l'environnement virtuel périphérique.

Les moyens de contrôle et de traitement peuvent par exemple contrôler les moyens de stimulation et les moyens de traitement des mots ou ensembles de mots.

Les moyens de contrôle et de traitement peuvent être avantageusement programmés pour commander le défilement du texte source à une vitesse de défilement sensiblement égale à la vitesse de lecture du texte par le sujet humain, déterminer si le sujet humain a lu le texte en entier, ou déterminer le temps de lecture du texte et/ou le nombre de mots lus par minute et/ou le nombre d'erreurs de lecture du texte.

Les moyens de traitement de mots peuvent être programmés pour
- analyser en temps réel les mots prononcés et captés en fonction des mots visualisés sur ledit parcours virtuel défilant (40),
- à chaque reconnaissance de mot, déterminer sa position dans le texte source,
- au premier mot reconnu, mettre à zéro un chronomètre interne,
- si le mot est trouvé dans le texte source, enregistrer le chronomètre pour ce mot et enregistrer l'état du mot reconnu.

Ces moyens de contrôle et de traitement peuvent en outre être programmés pour, à la fin du défilement du texte :
- calculer un taux de complétude du texte lu sur l'ensemble de ses mots,
- calculer un temps de lecture comme différence entre le dernier et le premier chronomètre enregistrés,
- afficher un tableau de résultat, affichant le texte, les mots reconnus en surbrillance, le taux de complétude et le temps de lecture.

Les moyens immersifs peuvent en outre être agencés pour commander en temps réel la visualisation d'un ensemble de mots et/ou de graphismes sur le parcours virtuel en fonction des données de tracking corporel et/ou de données de sortie des moyens de contrôle et de traitement.

Les moyens immersifs peuvent comprendre une structure de type Cave^{®} ou un mur immersif équipé d'un ou plusieurs vidéoprojecteurs 3D et une paire de lunettes 3D prévue pour être portée par le sujet humain.

Les moyens immersifs peuvent comprendre un casque de réalité virtuelle prévu pour être porté par le sujet humain et pour communiquer avec un dispositif de communication mobile.

Les moyens immersifs peuvent aussi comprendre une paire de lunettes de réalité augmentée prévue pour être portée par un sujet humain et pour communiquer avec un dispositif de communication mobile.

Dans un mode particulier de réalisation, le système de traitement selon l'invention met en œuvre un site local prévu pour recevoir le sujet humain équipé d'un moyen immersif portable et un site distant qui (i) est relié audit site local via un réseau de communication, (ii) comprend des moyens de contrôle et de traitement et (iii) est prévu pour recevoir un thérapeute équipé d'un second casque de réalité virtuelle.

Suivant un autre aspect il est proposé un procédé pour commander un système de traitement de troubles neurovisuels et/ou vestibulaires selon l'invention, comprenant les étapes suivantes :
- visualisation d'un ensemble de mots et/ou textes et/ou graphismes sur un parcours virtuel défilant dans un environnement interactif prévu pour recevoir un sujet humain en immersion,
- tracking d'une ou plusieurs parties du corps dudit sujet humain, pour délivrer des données de tracking corporel,
- captation sonore/vocale de mots ou ensemble de mots lus par ledit sujet humain en réponse aux mots ou texte visualisés sur le parcours virtuel défilant,
- traitement vocal de données de captation sonore de mots ou ensemble de mots prononcés par ledit sujet humain,

caractérisé en ce qu'il comprend en outre une étape pour commander en temps réel la visualisation en fonction des données de tracking corporel et des résultats de traitement des données de captation sonore, pour procurer audit sujet humain une visualisation simultanée (i) d'un environnement virtuel périphérique défilant à une première vitesse et (ii) d'un ensemble de mots d'un texte source sur un parcours virtuel défilant à une seconde vitesse,
et en ce qu'il comprend en outre des étapes de:
   - stimuler un ou plusieurs des sens dudit sujet humain, en vue de perturber sa lecture du texte source,
   - traiter les mots ou ensemble de mots prononcés et captés, de façon à délivrer des données représentatives de l'expérience de lecture du texte source par le sujet humain en relation avec des stimuli de perturbation émis par lesdits moyens de stimulation.

L'étape de stimulation peut avantageusement comprendre une émission d'un signal sonore perçu par le sujet humain, une modification de caractéristiques d'affichage du texte source, ou une visualisation d'un élément de distraction dans l'environnement virtuel périphérique.

Le défilement du texte source peut être avantageusement commandé à une vitesse sensiblement égale à la vitesse de lecture du texte par le sujet humain.

L'étape de traitement peut être agencée pour déterminer si le sujet humain a lu le texte en entier, ou déterminer le temps de lecture du texte et/ou le nombre de mots lus par minute et/ou le nombre d'erreurs de lecture du texte.

L'étape d'immersion peut en outre être programmée pour procurer au sujet humain une visualisation d'un environnement virtuel du parcours virtuel défilant, et pour commander spatialement le défilement d'un ensemble de mots et/ou de graphismes sur le parcours virtuel défilant, en fonction des données de tracking.

L'étape de tracking peut comprendre une captation des positions dans l'espace d'un ensemble de nœuds principaux du squelette du sujet humain.

L'étape d'immersion peut comprendre une étape pour calculer des points de vue du sujet humain en fonction de données de tracking dudit sujet humain et de caractéristiques d'un contenu visualisé sur le parcours virtuel défilant.

Le procédé de commande peut en outre comprendre une étape pour générer un fichier de grammaire spécifique à l'ensemble de mots et/ou textes et/ou graphismes issus d'un texte source et prévus pour être visualisés sur le parcours visuel défilant.

L'étape de traitement vocal peut comprendre une analyse en temps réel des mots lus par le sujet humain, ladite analyse comportant une reconnaissance parmi les mots lus de mots préalablement stockés dans le fichier de grammaire.

L'analyse temps réel des mots lus peut comprendre:
- pour chaque mot reconnu, une détermination de la position dudit mot reconnu dans le texte source,
- au premier mot reconnu, une initialisation d'un moyen de chronométrie,
- si le mot reconnu est trouvé dans le texte source, un enregistrement du temps mesuré par ledit moyen de chronométrie et de l'état dudit mot reconnu.

Le procédé de commande peut en outre comprendre, à la fin du défilement du texte :
- un calcul d'un taux de complétude du texte lu sur l'ensemble des mots,
- un calcul d'un temps de lecture sous la forme d'une différence entre la dernière et la première mesure de temps délivrés par le moyen de chronométrie,
- un affichage du texte et de résultats de traitement parmi un ensemble de résultats comprenant les mots reconnus, le taux de complétude et le temps de lecture.

Ainsi, avec le système de traitement selon l'invention, les orthoptistes bénéficient d'un nouvel outil thérapeutique mettant en œuvre une immersion ludique.

Ce système de traitement permet d'adresser les troubles de la vision périphérique, les saccades, les troubles de la lecture, les différentes dyslexies via un système simple et unique permettant de favoriser l'attention visuelle par la saturation visuelle périphérique en 3D.

Cette saturation visuelle périphérique permet d'améliorer la fluence en lecture, la précision des saccades dans un système multimodal (prise d'informations proprioceptives) chez les patients présentant un trouble des saccades oculomotrices qui sont souvent retrouvées dans les troubles du langage écrit (anciennement dyslexie mixte ou de surface ou visuo attentionnelle).

Suivant encore un autre aspect il est proposé un procédé pour traiter des troubles neurovisuels et/ou vestibulaires comprenant les étapes suivantes de :
- placer un sujet humain en immersion dans un environnement interactif visualisant un ensemble de mots et/ou textes et/ou graphismes sur un parcours virtuel défilant,
- collecter des données de tracking d'une ou plusieurs parties du corps dudit sujet humain,
- capter sous forme sonore/vocale des mots ou ensemble de mots lus par ledit sujet humain en réponse aux mots ou texte visualisés sur le parcours virtuel défilant,
- traiter des données de captation sonore de mots ou ensemble de mots prononcés par ledit sujet humain,
caractérisé en ce qu'il comprend en outre les étapes de :
- commander en temps réel la visualisation en fonction des données de tracking corporel et des résultats de traitement des données de captation sonore, pour procurer audit sujet humain une visualisation simultanée (i) d'un environnement virtuel périphérique défilant à une première vitesse et (ii) d'un ensemble de mots d'un texte source sur un parcours virtuel défilant à une seconde vitesse,
- stimuler un ou plusieurs des sens dudit sujet humain, en vue de perturber sa lecture du texte source,
- traiter les mots ou ensemble de mots prononcés et captés, de façon à délivrer des données représentatives de l'expérience de lecture du texte source par le sujet humain en relation avec des stimuli de perturbation émis par lesdits moyens de stimulation,
- à partir de ces données représentatives d'expérience, identifier un ou plusieurs troubles neurovisuels ou vestibulaires dudit sujet humain.

### DESCRIPTION DES FIGURES

On comprendra mieux l'invention en référence aux figures suivantes :
- La figure 1 illustre un premier exemple de réalisation d'un système de traitement selon l'invention, mettant en œuvre un équipement de type Cave^{®} ;
- La figure 2 illustre un second exemple de réalisation d'un système de traitement selon l'invention, mettant en œuvre des casques de réalité virtuelle ;
- La figure 3 illustre un troisième exemple de réalisation d'un système de traitement selon l'invention, mettant en œuvre un équipement de lunettes de réalité augmentée et un casque de réalité virtuelle ;
- La figure 4 est un schéma de principe d'un parcours virtuel défilant mis en œuvre dans un système de traitement selon l'invention ;
- La figure 5 illustre une copie d'écran de mots ou textes visualisés sur des routes défilantes et d'environnements virtuels ;
- La figure 6 illustre un exemple de processus de traitement mettant en œuvre le système de traitement selon l'invention.

### DESCRIPTION DETAILLEE

### Exemples de réalisation de systèmes de traitement

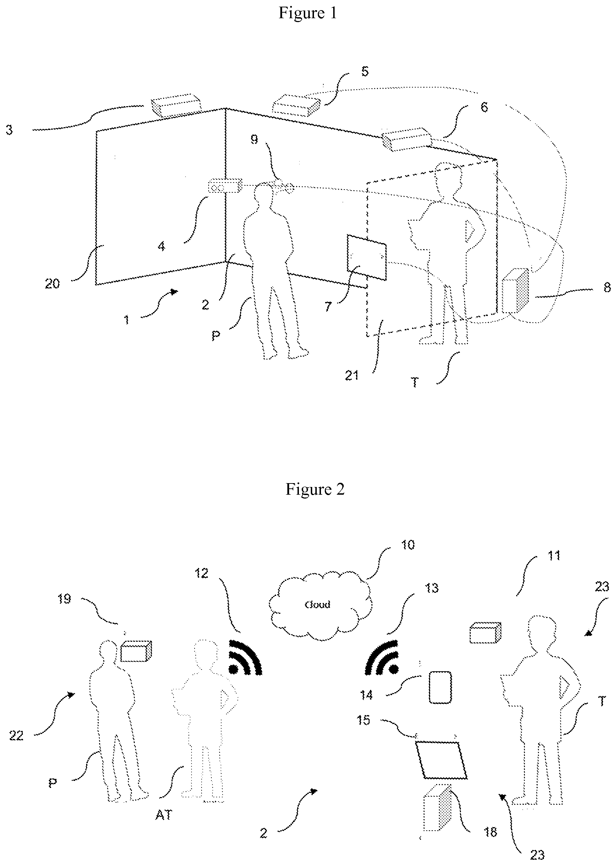

On va maintenant décrire, en référence à la Figure 1, un premier exemple de réalisation d'un système 1 de traitement de troubles neurovisuels. Ce système de traitement 1 met en œuvre une structure Cave ^{®} comportant trois parois - une paroi centrale 2 et deux parois latérales optionnelles - et équipée d'un vidéoprojecteur 3D par paroi. Le système de traitement 1 comprend en outre une caméra de profondeur 4, une paire 9 de lunettes 3D confiée à un sujet humain ou patient P, un ordinateur 8 et un écran tactile de contrôle 7.

Les trois vidéoprojecteurs 3,5,6 sont commandés par l'ordinateur 8 pour réaliser une immersion du patient P équipé des lunettes 3D dans un environnement virtuel comportant un parcours virtuel défilant. La caméra de profondeur 4 positionnée pour suivre les mouvements du corps du patient P délivre des données de tracking qui sont transmises à l'ordinateur 8. Un thérapeute ou praticien T pilote le système de traitement 1 au moyen de l'écran tactile 7 qui est relié à l'ordinateur 8.

Dans un second exemple de réalisation illustré par la Figure 2, le système de traitement 2 comprend, sur un site local 22, un casque de réalité virtuelle 19 prévu pour équiper un patient P et pour communiquer via une liaison sans fil locale 12, un serveur informatique cloud 10 et une liaison sans fil distante 13 avec un site distant 23. Le système de traitement 2 comprend en outre sur ce site distant 23 un ordinateur 18 implémentant un programme de contrôle du système de traitement 2, un écran tactile 15 permettant à un thérapeute T de piloter ce système 2, un terminal mobile 14 et un casque de réalité virtuelle autonome 11 équipant ce thérapeute T. Depuis le site distant 23, le thérapeute T peut initier une session de traitement à partir de l'écran tactile 15. Des instructions et du flux vidéo immersif sont transmis vers le site local 22 via le terminal mobile 14, les moyens de liaison sans fil 13,12 et le serveur informatique cloud 10 jusqu'au casque de réalité virtuelle 19 équipant le patient P. On peut ainsi prévoir que le patient P puisse visualiser dans son casque 19 d'une part un avatar AT du thérapeute T et d'autre part un environnement virtuel dynamique incluant un parcours virtuel défilant sur lequel un texte ou un ensemble de mots est visualisé.

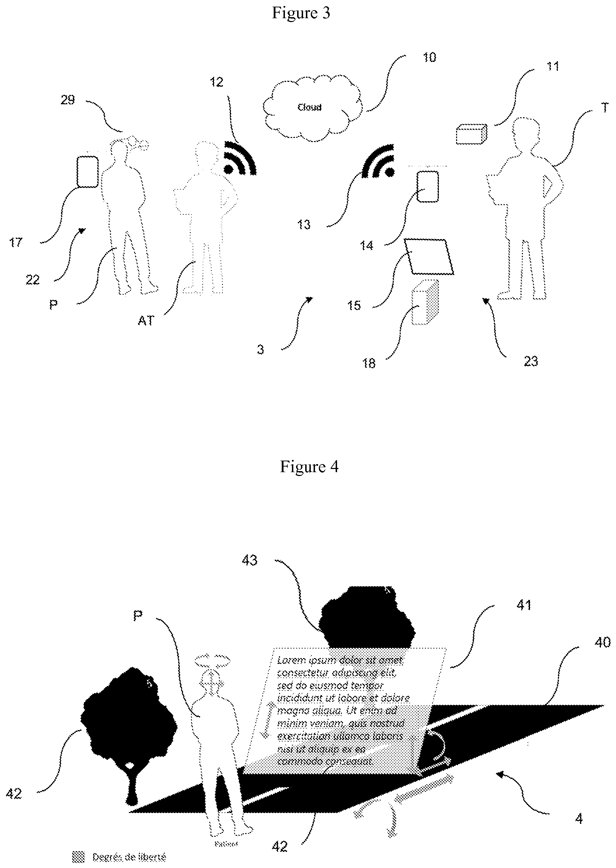

Dans un troisième exemple de réalisation d'un système de traitement 5 selon l'invention illustré par la Figure 3, le patient P situé sur le site local 22 est équipé d'un dispositif de lunettes de réalité augmentée 29 communiquant avec un terminal mobile 17 lui-même connecté via la liaison WiFi locale 12, le serveur informatique cloud 10 et la liaison WiFi distante 13 avec le terminal mobile 14 équipant le thérapeute T et l'ordinateur 18 pilotant le système 2.

Quel que soit le mode d'immersion - Cave^{®}, mur immersif, casque de réalité virtuelle ou lunettes de réalité augmentée -, le système de traitement selon l'invention peut être programmé pour immerger un patient P dans un environnement virtuel immersif tel que représenté schématiquement en Figure 4.

Cet environnement virtuel immersif 4 comprend un parcours virtuel défilant 40 sur lequel est affiché un texte ou un ensemble de mots 41 et un environnement virtuel périphérique incluant par exemple des arbres 42,43 ou d'autres éléments représentés virtuellement. Le parcours virtuel 40 défile à une première vitesse tandis que l'environnement virtuel périphérique 42,43 défile à une seconde vitesse qui peut être différente de la première vitesse.

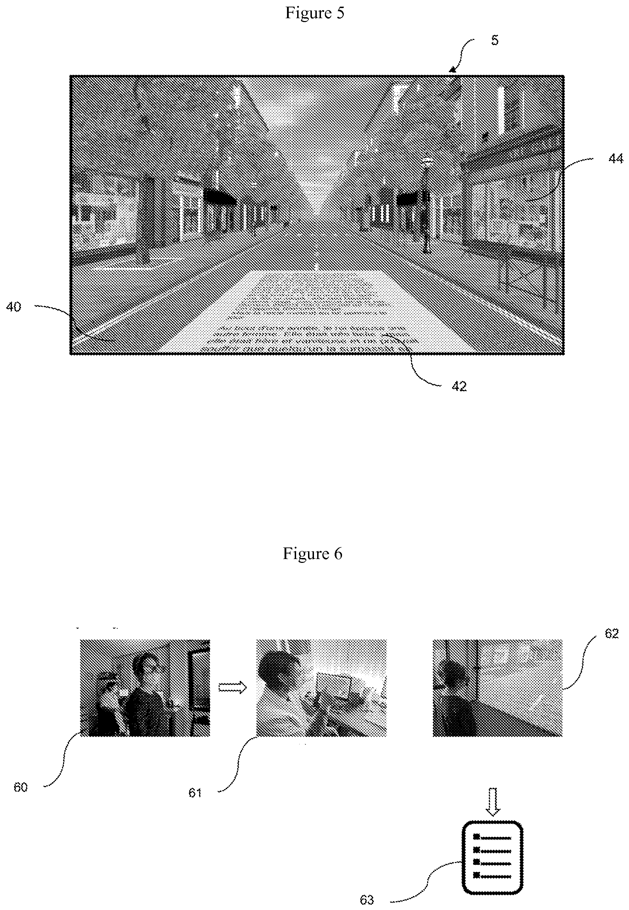

L'environnement virtuel immersif 4 implémente plusieurs degrés de liberté à la fois pour le patient P qui peut déplacer sa tête, pour le parcours virtuel défilant 40 et pour les textes projetés qui peuvent présenter une inclinaison et une orientation réglables. Ainsi, en référence à la Figure 5, le patient P peut visualiser par exemple sur un écran immersif 5 un texte défilant 42 sur une route virtuelle 40.

### Exemple de mise en œuvre d'un système de traitement

On va maintenant décrire, en référence à la Figure 6, un exemple d'une session de traitement mettant en œuvre le système de traitement 1 illustré en Figure 1. Dans une première étape 60, le patient P est équipé d'un dispositif de vision 3D lui permettant d'être immergé dans un environnement virtuel. Le thérapeute T paramètre la session: changement du texte, des paramètres de défilement de ce texte et d'un décor virtuel (étape 61).

Le thérapeute programme alors un ensemble de stimuli de perturbation sonores et/ou visuels qui vont interférer dans la lecture du texte par le sujet ou patient et vont permettre de détecter et de caractériser, après traitement vocal, d'éventuels troubles de la lecture ou de l'attention. A titre d'exemples non limitatifs, des stimuli sonores peuvent comprendre des bruits de klaxon, des cris ou des nuisances sonores urbaines. Des stimuli visuels peuvent comprendre l'apparition brutale d'un personnage ou d'un véhicule dans l'environnement virtuel périphérique, ou bien un changement soudain de la police de caractères utilisée pour l'affichage du texte source.

Le patient P lit alors à haute voix le texte affiché à l'écran (étape 62). Un dispositif de captation sonore capte la voix du patient P qui est analysée et traitée par un programme de traitement vocal installé sur l'ordinateur 8. Les résultats d'analyse sont ensuite enregistrés et historisés dans une unité de mémoire locale ou distante (étape 63).

Le système de traitement selon l'invention peut être configuré pour effectuer les traitement automatisés suivants :
- capture, lissage et extrapolation à 60Hz, au moyen d'un algorithme dédié, des positions dans l'espace des principaux nœuds de squelette de l'utilisateur (le capteur de profondeur utilisé ne mesure qu'à 30Hz avec des imprécisions),
- calcul du point de vue de l'utilisateur, génération d'une image par œil de l'utilisateur en fonction des paramètres de position du patient utilisateur et de contenu virtuel souhaité (inclinaison texte, changement polices, couleurs, transparence, vitesse de défilement)
- archivage du résultat de la séance, intégrant : paramètres d'entrée pris en compte, résultat de lecture (automatisé ou saisi manuellement par le thérapeute)

### Traitement automatisé de reconnaissance vocale

Un exemple pratique de traitement automatisé vocal mis en œuvre dans le système de traitement selon l'invention est le suivant :
1. Au lancement de la séance : le système de traitement prépare le module de reconnaissance vocale via la génération automatique d'un fichier de grammaire spécifique au texte qui va être affiché à l'utilisateur
2. Durant le défilement du texte :
   - le sujet ou patient lit à haute voix le texte
   - le système analyse en temps réel les mots lus par l'utilisateur
      - à chaque reconnaissance de mot, détermination de sa position dans le texte source
      - au premier mot reconnu : mise à zéro d'un chronomètre interne
      - si le mot est trouvé dans le texte source :
         - enregistrement du chronomètre pour ce mot
         - enregistrement de l'état du mot « reconnu »
3. lorsque le texte a terminé son défilement
   - calcul d'un taux de complétude du texte lu sur l'ensemble de ses mots
   - calcul du temps de lecture comme différence entre dernier et premier chronomètre enregistré
   - affichage d'un tableau de résultat, affichant le texte, les mots reconnus en surbrillance, le taux de complétude et le temps de lecture
4. archivage du résultat de lecture dans la base de données « patient ».

Bien entendu, l'invention n'est pas limitée aux exemples qui viennent d'être décrits et de nombreux autres modes de réalisation peuvent être envisagés sans sortir du cadre de la présente invention. Ainsi, d'autres techniques immersives pourront être envisagées en cas d'évolutions technologiques notables dans ce domaine. Par ailleurs, les textes prévus pour être affichés sur le parcours virtuel défilant ne sont en aucun limités à ceux représentés sur les figures 4 ou 5 et peuvent être librement choisis par le thérapeute opérant le système de traitement. Le champ d'application de la présente invention est défini par les revendications ci-jointes.

### REFERENCES

**1.** A. BULLINGER, Le développement sensori-moteur de l'enfant et ses avatars, Ed. ERES, 2004.
**2.** Mémoire en vue de l'obtention du certificat de capacité d'orthophonie, présenté par Chloé Teysseyre, juin 2012.

## Revendications

1. Système (1-3) pour traiter des troubles neurovisuels et/ou vestibulaires présentés par un sujet humain (P), comprenant :
- des moyens immersifs (2,9,19,29) prévus pour immerger ledit sujet humain (P) dans un environnement interactif procurant une restitution stéréoscopique, agencés pour procurer au sujet humain (P) une saturation de la vision périphérique,
- des moyens de tracking (4) d'une ou plusieurs parties du corps dudit sujet humain (P), pour délivrer des données de tracking corporel,
- des moyens de contrôle et de traitement (8,18), prévus pour piloter lesdits moyens immersifs (2,9,19,29), lesdits moyens de contrôle et de traitement exploitant les données de tracking corporel pour procurer audit sujet humain (P) une visualisation simultanée (i) d'un environnement virtuel périphérique (42,44) défilant à une première vitesse et (ii) d'un ensemble de mots (42) d'un texte source sur un parcours virtuel (40) défilant à une seconde vitesse
- des moyens de captation sonore, prévus pour capter des mots ou ensemble de mots prononcés par ledit sujet humain (P),
- des moyens pour émettre des stimuli supplémentaires programmés de perturbation sonores et/ou visuels prévus pour interférer dans la lecture dudit texte par ledit sujet humain,
- des moyens pour traiter les mots ou ensemble de mots prononcés et captés, de façon à délivrer des données représentatives de l'expérience de lecture du texte source par le sujet humain en relation avec des stimuli de perturbation émis par lesdits moyens de stimulation.
où les moyens de traitement sont programmés pour
- analyser en temps réel les mots prononcés et captés en fonction des mots visualisés sur ledit parcours virtuel défilant (40),
- à chaque reconnaissance de mot, déterminer sa position dans le texte source,
- au premier mot reconnu, mettre à zéro un chronomètre interne,
- pour chaque mot parmi les mots prononcés et reconnus trouvé dans le texte source, enregistrer le chronomètre pour ce mot et enregistrer l'état du mot reconnu.

2. Système de traitement selon la revendication précédente, **caractérisé en ce que** les moyens de contrôle et de traitement sont en outre programmés pour, à la fin du défilement du texte :
- calculer un taux de complétude du texte lu sur l'ensemble de ses mots,
- calculer un temps de lecture comme différence entre le dernier et le premier chronomètre enregistrés,
afficher un tableau de résultat, affichant le texte, les mots reconnus en surbrillance, le taux de complétude et le temps de lecture

3. Système de traitement selon l'une des revendications précédentes, **caractérisé en ce que** les stimuli de perturbation comprennent une émission d'un signal sonore perçu par le sujet humain.

4. Système de traitement selon l'une quelconque des revendications précédentes, **caractérisé en ce que** les stimuli de perturbation comprennent une modification de caractéristiques d'affichage du texte source.

5. Système de traitement selon l'une quelconque des revendications précédentes, **caractérisé en ce que** les stimuli de perturbation comprennent une visualisation d'un élément de distraction dans l'environnement virtuel périphérique.

6. Système de traitement selon l'une quelconque des revendications précédentes, **caractérisé en ce que** les moyens de contrôle et de traitement contrôlent les moyens de stimulation et les moyens de traitement des mots ou ensembles de mots.

7. Système de traitement selon l'une quelconque des revendications précédentes, **caractérisé en ce que** les moyens de contrôle et de traitement sont programmés pour commander le défilement du texte source à une vitesse de défilement sensiblement égale à la vitesse de lecture du texte par le sujet humain.

8. Système de traitement selon l'une quelconque des revendications précédentes, **caractérisé en ce que** les moyens de traitement sont programmés pour déterminer si le sujet humain a lu le texte en entier.

9. Système de traitement selon l'une quelconque des revendications précédentes, **caractérisé en ce que** les moyens de traitement sont programmés pour déterminer le temps de lecture du texte et/ou le nombre de mots lus par minute et/ou le nombre d'erreurs de lecture du texte.

10. Système de traitement (1) selon l'une quelconque des revendications précédentes, **caractérisé en ce que** les moyens immersifs (1) comprennent une structure de type CAVE ( ou un mur immersif) équipée d'un ou plusieurs vidéoprojecteurs 3D (3,5,6) et une paire de lunettes 3D (9) prévue pour être portée par le sujet humain (P).

11. Système de traitement (2) selon l'une quelconque des revendications 1 à 9, **caractérisé en ce que** les moyens immersifs comprennent un casque de réalité virtuelle (19) prévu pour être porté par le sujet humain (P) et pour communiquer avec un dispositif de communication mobile (14).

12. Système de traitement (3) selon l'une quelconque des revendications 1 à 9, **caractérisé en ce que** les moyens immersifs comprennent une paire de lunettes de réalité augmentée (9) prévue pour être portée par le sujet humain (P) et pour communiquer avec un dispositif de communication mobile (17).

13. Système de traitement (2,3) selon l'une quelconque des revendications 1 à 9, **caractérisé en ce qu'**il comprend un moyen immersif portable (19,26) équipant le sujet humain (P) dans un site local (22) relié à un site distant (23) via un réseau de communication (12,10,13), et dans ledit site distant (23), des moyens de contrôle de traitement (19) et un casque de réalité virtuelle (11) équipant un thérapeute.

## Patentansprüche

1. System (1-3) zur Behandlung neurovisueller und/oder vestibulärer Störungen, die bei einem menschlichen Subjekt (P) auftreten, umfassend:
- Immersionsmittel (2, 9, 19, 29), die dazu vorgesehen sind, das menschliche Subjekt (P) in eine interaktive Umgebung einzutauchen, die eine stereoskopische Wiedergabe bereitstellt, und die dazu angeordnet sind, dem menschlichen Subjekt (P) eine Sättigung des peripheren Sehens zu verschaffen;
- Tracking-Mittel (4) für einen oder mehrere Körperteile des menschlichen Subjekts (P), um Körper-Tracking-Daten bereitzustellen;
- Steuerungs- und Verarbeitungsmittel (8, 18), die dazu vorgesehen sind, die Immersionsmittel (2, 9, 19, 29) zu steuern, wobei die Steuerungs- und Verarbeitungsmittel die Körper-Tracking-Daten nutzen, um dem menschlichen Subjekt (P) eine gleichzeitige Visualisierung (i) einer peripheren virtuellen Umgebung (42 44), die mit einer ersten Geschwindigkeit scrollt, und (ii) einer Gruppe von Wörtern (42) eines Ausgangstextes auf einem virtuellen Pfad (40), der mit einer zweiten Geschwindigkeit scrollt, bereitzustellen;
- Tonerfassungsmittel, die dazu vorgesehen sind, von dem menschlichen Subjekt (P) gesprochene Wörter oder Wortgruppen zu erfassen;
- Stimulationsmittel, die dazu vorgesehen sind, programmierte zusätzliche akustische und/oder visuelle Störreize auszusenden, die das Lesen des Textes durch das menschliche Subjekt stören sollen;
- Mittel zum Verarbeiten der gesprochenen und erfassten Wörter oder Wortgruppen, um Daten bereitzustellen, die die Leseerfahrung des menschlichen Subjekts mit dem Ausgangstext in Bezug auf von den Stimulationsmitteln ausgesendete Störreize darstellen,
wobei die Verarbeitungsmittel dazu programmiert sind:
- die gesprochenen und erfassten Wörter in Abhängigkeit von den auf dem scrollenden virtuellen Pfad (40) visualisierten Wörtern in Echtzeit zu analysieren;
- bei jeder Worterkennung dessen Position im Ausgangstext zu bestimmen;
- beim ersten erkannten Wort einen internen Zeitgeber auf null zurückzusetzen;
- für jedes Wort unter den gesprochenen und erkannten Wörtern, das im Ausgangstext gefunden wird, den Zeitgeberwert für dieses Wort und den Status des erkannten Wortes zu speichern.

2. Behandlungssystem nach dem vorhergehenden Anspruch, **dadurch gekennzeichnet, dass** die Steuerungs- und Verarbeitungsmittel ferner dazu programmiert sind, am Ende des Scrollens des Textes:
- einen Vollständigkeitsgrad des gelesenen Textes über sämtliche Wörter zu berechnen;
- eine Lesezeit als Differenz zwischen dem letzten und dem ersten gespeicherten Zeitgeberwert zu berechnen;
- eine Ergebnistabelle anzuzeigen, in der der Text, die hervorgehobenen erkannten Wörter, der Vollständigkeitsgrad und die Lesezeit angezeigt werden.

3. Behandlungssystem nach einem der vorhergehenden Ansprüche, **dadurch gekennzeichnet, dass** die Störreize das Aussenden eines von dem menschlichen Subjekt wahrgenommenen Tonsignals umfassen.

4. Behandlungssystem nach einem der vorhergehenden Ansprüche, **dadurch gekennzeichnet, dass** die Störreize eine Änderung von Anzeigeeigenschaften des Ausgangstextes umfassen.

5. Behandlungssystem nach einem der vorhergehenden Ansprüche, **dadurch gekennzeichnet, dass** die Störreize eine Visualisierung eines Ablenkungselements in der peripheren virtuellen Umgebung umfassen.

6. Behandlungssystem nach einem der vorhergehenden Ansprüche, **dadurch gekennzeichnet, dass** die Steuerungs- und Verarbeitungsmittel die Stimulationsmittel und die Mittel zum Verarbeiten der Wörter oder Wortgruppen steuern.

7. Behandlungssystem nach einem der vorhergehenden Ansprüche, **dadurch gekennzeichnet, dass** die Steuerungs- und Verarbeitungsmittel dazu programmiert sind, das Scrollen des Ausgangstextes mit einer Scrollgeschwindigkeit zu steuern, die im Wesentlichen gleich der Lesegeschwindigkeit des Textes durch das menschliche Subjekt ist.

8. Behandlungssystem nach einem der vorhergehenden Ansprüche, **dadurch gekennzeichnet, dass** die Verarbeitungsmittel dazu programmiert sind, zu bestimmen, ob das menschliche Subjekt den Text vollständig gelesen hat.

9. Behandlungssystem nach einem der vorhergehenden Ansprüche, **dadurch gekennzeichnet, dass** die Verarbeitungsmittel dazu programmiert sind, die Lesezeit des Textes und/oder die Anzahl der pro Minute gelesenen Wörter und/oder die Anzahl der Lesefehler im Text zu bestimmen.

10. Behandlungssystem (1) nach einem der vorhergehenden Ansprüche, **dadurch gekennzeichnet, dass** die Immersionsmittel (1) eine CAVE-artige Struktur (oder eine immersive Wand) umfassen, die mit einem oder mehreren 3D-Videoprojektoren (3, 5, 6) und einer 3D-Brille (9) ausgestattet ist, die dazu vorgesehen ist, von dem menschlichen Subjekt (P) getragen zu werden.

11. Behandlungssystem (2) nach einem der Ansprüche 1 bis 9, **dadurch gekennzeichnet, dass** die Immersionsmittel ein Virtual-Reality-Headset (19) umfassen, das dazu vorgesehen ist, von dem menschlichen Subjekt (P) getragen zu werden und mit einer mobilen Kommunikationsvorrichtung (14) zu kommunizieren.

12. Behandlungssystem (3) nach einem der Ansprüche 1 bis 9, **dadurch gekennzeichnet, dass** die Immersionsmittel eine Augmented-Reality-Brille (9) umfassen, die dazu vorgesehen ist, von dem menschlichen Subjekt (P) getragen zu werden und mit einer mobilen Kommunikationsvorrichtung (17) zu kommunizieren.

13. Behandlungssystem (2, 3) nach einem der Ansprüche 1 bis 9, **dadurch gekennzeichnet, dass** es ein tragbares Immersionsmittel (19, 26), mit dem das menschliche Subjekt (P) an einem lokalen Standort (22) ausgestattet ist, der über ein Kommunikationsnetzwerk (12, 10, 13) mit einem entfernten Standort (23) verbunden ist, sowie an dem entfernten Standort (23) Behandlungssteuerungsmittel (19) und ein Virtual-Reality-Headset (11), mit dem ein Therapeut ausgestattet ist, umfasst.

## Claims

1. A system (1-3) for treating neurovisual and/or vestibular disorders exhibited by a human subject (P), comprising:
- immersive means (2, 9, 19, 29) configured to immerse said human subject (P) in an interactive environment providing stereoscopic rendering, arranged to provide the human subject (P) with saturation of peripheral vision;
- tracking means (4) for one or more parts of the body of said human subject (P), for providing body-tracking data;
- control and processing means (8, 18), configured to control said immersive means (2, 9, 19, 29), said control and processing means using the body-tracking data to provide said human subject (P) with simultaneous visualisation of (i) a peripheral virtual environment (42 44) scrolling at a first speed and (ii) a set of words (42) of a source text on a virtual path (40) scrolling at a second speed;
- sound-capture means configured to capture words or sets of words spoken by said human subject (P);
- stimulation means configured to emit programmed additional audible and/or visual disturbance stimuli intended to interfere with the reading of said text by said human subject;
- means for processing the spoken and captured words or sets of words so as to provide data representative of the human subject's reading experience of the source text in relation to disturbance stimuli emitted by said stimulation means,
wherein the processing means are programmed to:
- analyse in real time the spoken and captured words as a function of the words displayed on said scrolling virtual path (40);
- upon each word recognition, determine its position in the source text;
- upon the first recognised word, reset an internal timer to zero;
- for each word among the spoken and recognised words found in the source text, record the timer reading for that word and record the status of the recognised word.

2. The treatment system according to the preceding claim, **characterised in that** the control and processing means are further programmed, at the end of scrolling of the text, to:
- calculate a completion rate of the text read over all of its words;
- calculate a reading time as the difference between the last and first recorded timer readings;
- display a results table showing the text, the recognised words highlighted, the completion rate and the reading time.

3. The treatment system according to any one of the preceding claims, **characterised in that** the disturbance stimuli comprise emission of a sound signal perceived by the human subject.

4. The treatment system according to any one of the preceding claims, **characterised in that** the disturbance stimuli comprise modification of display characteristics of the source text.

5. The treatment system according to any one of the preceding claims, **characterised in that** the disturbance stimuli comprise visualisation of a distracting element in the peripheral virtual environment.

6. The treatment system according to any one of the preceding claims, **characterised in that** the control and processing means control the stimulation means and the means for processing the words or sets of words.

7. The treatment system according to any one of the preceding claims, **characterised in that** the control and processing means are programmed to control scrolling of the source text at a scrolling speed substantially equal to the reading speed of the text by the human subject.

8. The treatment system according to any one of the preceding claims, **characterised in that** the processing means are programmed to determine whether the human subject has read the text in full.

9. The treatment system according to any one of the preceding claims, **characterised in that** the processing means are programmed to determine the reading time of the text and/or the number of words read per minute and/or the number of reading errors in the text.

10. The treatment system (1) according to any one of the preceding claims, **characterised in that** the immersive means (1) comprise a CAVE-type structure (or an immersive wall) equipped with one or more 3D video projectors (3, 5, 6) and a pair of 3D glasses (9) configured to be worn by the human subject (P).

11. The treatment system (2) according to any one of claims 1 to 9, **characterised in that** the immersive means comprise a virtual-reality headset (19) configured to be worn by the human subject (P) and to communicate with a mobile communication device (14).

12. The treatment system (3) according to any one of claims 1 to 9, **characterised in that** the immersive means comprise a pair of augmented-reality glasses (9) configured to be worn by the human subject (P) and to communicate with a mobile communication device (17).

13. The treatment system (2, 3) according to any one of claims 1 to 9, **characterised in that** it comprises a portable immersive means (19, 26) equipping the human subject (P) at a local site (22) connected to a remote site (23) via a communication network (12, 10, 13), and, at said remote site (23), treatment control means (19) and a virtual-reality headset (11) equipping a therapist.
